(19) Europäisches Patentamt
European Patent Office
Office européen des brevets

(11) EP 2 404 601 A1

# (12) EUROPEAN PATENT APPLICATION

(43) Date of publication:
**11.01.2012 Bulletin 2012/02**

(21) Application number: **10168641.8**

(22) Date of filing: **06.07.2010**

(51) Int Cl.:
***A61K 31/496*** *(2006.01)* ***A61K 31/664*** *(2006.01)*
***A61P 33/00*** *(2006.01)* ***A61P 33/06*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **BioAgency AG**
**22525 Hamburg (DE)**

(72) Inventors:
- **Gutteridge, Winston**
  **Sevenoaks**
  **Kent TN13 1QD (GB)**
- **Hutchinson, David**
  **Westerham**
  **Kent, TN16 2DR (GB)**

(74) Representative: **Simandi, Claus**
**Patentanwalt**
**Höhenstrasse 26**
**53773 Hennef / Bonn (DE)**

## (54) New drug combinations for the treatment of Malaria

(57) The present invention relates to pharmaceutical preparations / compositions comprising 3-N-formyl hydroxy amino propyl phosphonic acid derivatives or 3-N-acetyl hydroxy amino propyl phosphonic acid derivatives as active ingredients in combination with Piperaquine.

EP 2 404 601 A1

Printed by Jouve, 75001 PARIS (FR)

## Description

The present invention relates to pharmaceutical preparations / compositions comprising 3-N-formyl hydroxy amino propyl phosphonic acid derivatives or 3-N-acetyl hydroxy amino propyl phosphonic acid derivatives as active ingredients in combination with Piperaquine.

The use of 3-N-formyl hydroxy amino propyl phosphonic acid derivatives and 3-N-acetyl hydroxy amino propyl phosphonic acid derivatives for prophylactic and therapeutic treatment of infectious processes, especially infections caused by unicellular parasites (with the meaning of this invention: protozoa) or multicellular parasites, is already known from DE19825585. A bacterial activity has already been described in DE2733658.

Additional, EP1071409B1 describes the use of 3-N-formyl hydroxy amino propyl phosphonic acid derivatives and 3-N-acetyl hydroxy amino propyl phosphonic acid derivatives for the treatment of infectious processes, especially infections caused by unicellular parasites (with the meaning of this invention: protozoa) or multicellular parasites.

Moreover, the use of 3-N-formyl hydroxy amino propyl phosphonic acid derivatives and 3-N-acetyl hydroxy amino propyl phosphonic acid derivatives in combination with one special pharmaceutical active ingredient for prophylactic and therapeutic treatment of infectious processes, especially infections caused by unicellular parasites (with the meaning of this invention: protozoa) or multicellular parasites, is already known from EP1305031. Furthermore, EP1305031 discloses preferred and selected combination with compounds like clindamycin (CDN) and azithromycin (AZT).

Fosmidomycin (FOS, 3-[Formyl(hydroxy)amino]propoylphosphonic acid, see below) exerts its anti-malarial activity by inhibition of 1-deoxy-D-xylulose 5-phosphate (DOXP) isomerase (Jomaa, H., J. Wiesner, S. Sanderbrand, B. Altincicek, C. Weidemeyer, M. Hintz, I. Turbachova, M. Eberl, J. Zeidler, H. K. Lichtenthaler, D. Soldati, and E. Beck. 1999. Inhibitors of the nonmevalonate pathway of isoprenoid biosynthesis as antimalarial drugs. Science 285:1573-6), an enzyme present in the Plasmodium apicoplast but absent from humans. Clinical trials established the drug's ability to rapidly clear parasites and reduce fever but recrudescence rates were high (Lell, B., R. Ruangweerayut, J. Wiesner, M. A. Missinou, A. Schindler, T. Baranek, M. Hintz, D. Hutchinson, H. Jomaa, and P. G. Kremsner. 2003. Fosmidomycin, a novel chemotherapeutic agent for malaria. Antimicrob Agents Chemother 47:735-8).

Further trials using combinations of Fosmidomycin and Clindamycin or Artesunate increased cure rates in comparison to Fosmidomycin alone (Borrmann, S., A. A. Adegnika, F. Moussavou, S. Oyakhirome, G. Esser, P. B. Matsiegui, M. Ramharter, 1. Lundgren, M. Kombila, S. Issifou, D. Hutchinson, J. Wiesner, H. Jomaa, and P. G. Kremsner. 2005. Short-course regimens of artesunate-fosmidomycin in treatment of uncomplicated Plasmodium falciparum malaria. Antimicrob Agents Chemother 49:3749-54; Borrmann, S., S. Issifou, G. Esser, A. A. Adegnika, M. Ramharter, P. B. Matsiegui, S. Oyakhirome, D. P. Mawili-Mboumba, M. A. Missinou, J. F. Kun, H. Jomaa, and P. G. Kremsner. 2004. Fosmidomycin-clindamycin for the treatment of Plasmodium falciparum malaria. J Infect Dis 190:1534-40). The increasing evidence of reduced artesunate efficacy (Noedl, H., Y. Se, K. Schaecher, B. L. Smith, D. Socheat, and M. M. Fukuda. 2008. Evidence of artemisinin-resistant malaria in western Cambodia. N Engl J Med 359:2619-20) has lead to the reconsideration of non-artemisinin-based combinations.

Piperaquine (*PIP,* see below) is well known for the treatment of malaria. Piperaquine is a bisquinoline first synthesized in the 1960s, and used extensively in China and Indochina as prophylaxis and treatment of malaria. Usage declined in the 1980s as piperaquine-resistant strains of P. falciparum arose and artemisinin-based antimalarials became available (e.g. Dihydroartemisinin-piperaquine (Eurartesim)).

However, there is a large need to provide a novel stable combination maintaining the activity of Piperaquine *(PIP)* due to the fact that the infectious agents become drug-resistant.

Even if all these compounds exhibit good results in the treatment of infections caused by parasites or bacteria, also these medicaments exhibit undesired side-effects and there is an ongoing need to improve the efficacy of the drug. Hence, there is a large need to optimize and identify a drug combination having an improved anti-malaria efficacy.

Therefore, the present invention relates to the object to enhance activity, stability and efficacy of a pharmaceutical preparation without increasing the side-effects of these active ingredients. Pharmaceutical preparations shall be made available providing a reduction of side-effects and improvement of efficacy. The object is as well to widen the range or therapeutic application of said pharmaceutical preparations and especially also to extend it to the treatment of problematic groups such as children and pregnant women. The anti-parasitic activity shall be increased to such a degree such that these pharmaceutical preparations may be administered in lower doses and, thus, a reduction or elimination of side effects caused by these preparations is achieved. Moreover, the very preferred optimization shall be effected for the prophylaxis and treatment of malaria and / or toxoplasmosis.

Surprisingly, it has been found that 3-N-formyl hydroxy amino propyl phosphonic acid derivatives and 3-N-acetyl hydroxy amino propyl phosphonic acid derivatives in combination with one or more further pharmaceutical preparations / compounds resulting in a combination with

Piperaquine

and optionally

being selected from a further second group consisting of
clindamycin, lincomycin, mirincamycin, pirlimycin and other lincosamides; minocycline and other tetracycline derivatives, or
azithromycin, erythromycin, spiramycin, josamycin, roxithromycin, clarithromycin, midecamycin and other macrolide antibiotics.'
or
artemisinin, Dihydroartemisinin
are especially suited for the treatment of malaria and / or toxoplasmosis.

According to the present invention 3-N-formyl-hydroxy amino propyl phosphonic acid derivatives and 3-N-acetyl hydroxy amino propyl phosphonic acid derivatives are deemed to be compounds of formula (I)

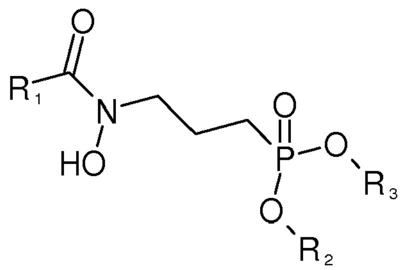

wherein R.sub.1 is selected from the group consisting of hydrogen and methyl, and
wherein R.sub.2 and R.sub.3 are independently selected from the group, consisting of hydrogen, substituted or unsubstituted alkyl, substituted or unsubstituted acyl, substituted or unsubstituted aryl, substituted or unsubstituted aralkyl, substituted or unsubstituted cycloalkyl, substituted or unsubstituted silyl, substituted or unsubstituted heterocyclic residue, or together form a substituted or unsubstituted C.sub.1-5-alkyl chain, the alkyl groups being saturated or comprising one or more double bonds or triple bonds.

However, in a preferred embodiment of the invention the formula I is fosmydomycin, (FOS) wherein R.sub.1 is hydrogen and R.sub.2 and R.sub.3 are hydrogen or a salt thereof, in particular 3-[Formyl(hydroxy)amino]propoylphosphonic acid (CAS No. 66508-53-0).

Piperaquine (PIP) is 7-chloro-4-[4-[3-[4-(7-chloroquinolin-4-yl)piperazin-1-yl]propyl] piperazin-1-yl]quinoline (CAS No. 4085-31-8) represented by formula (II)

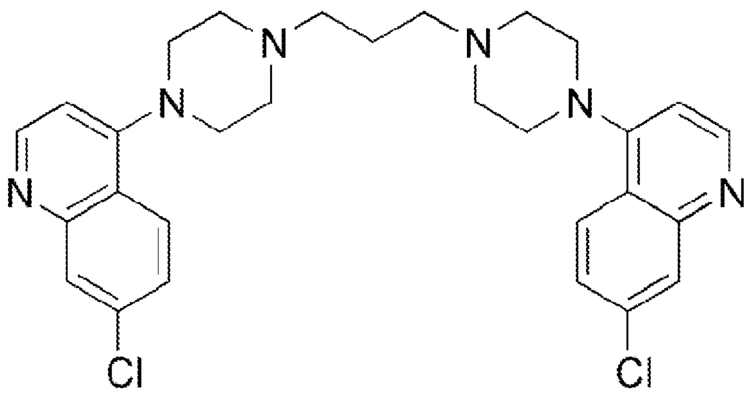

The combination preparations in accordance with the invention are also deemed to be the respective salts (e.g. Natrium-salts or the like).

Special features of the above definitions and suitable examples thereof are stated below:

"Acyl" is a substituent which originates from an acid, such as from an organic carboxylic acid, carbonic acid, carbamic acid or the thioacid or imidic acid corresponding to the individual above-stated acids, or from an organic sulfonic acid, wherein these acids may in each case comprise aliphatic, aromatic and/or heterocyclic groups in the molecule, as well as carbamoyl or carbamimidoyl.

Suitable examples of these acyl groups are stated below.

Aliphatic acyl groups are deemed to comprise acyl residues originating from an aliphatic acid, such groups including the following:

alkanoyl (for example formyl, acetyl, propionyl, butyryl, isobutyryl, valeryl, isovaleryl, pivaloyl etc.);
alkenoyl (for example acryloyl, methacryloyl, crotonoyl etc.);
alkylthioalkanoyl (for example methylthioacetyl, echylthioacetyl etc.);
alkanesulfonyl (for example mesyl, echanesulfonyl, propanesulfonyl etc.);
alkoxycarbonyl (for example methoxycarbonyl, ethoxycarbonyl, propoxycarbonyl, isopropoxycarbonyl, butoxycarbonyl, isobutoxycarbonyl etc.);
alkylcarbamoyl (for example methylcarbamoyl etc.);
(N-alkyl)thiocarbamoyl (for example (N-methyl)thiocarbamoyl etc.); alkylcarbamimidoyl (for example methylcarbamimidoyl etc.);
oxalo;
alkoxalyl (for example methoxalyl, ethoxalyl, propoxalyl etc.).

In the above examples of aliphatic acyl groups, the aliphatic hydrocarbon moiety, in particular the alkyl group or alkane residue, may optionally comprise one or more suitable substituents, such as amino, halogen (for example fluorine, chlorine, bromine etc.), hydroxy, hydroxyimino, carboxy, alkoxy (for example methoxy, ethoxy, propoxy etc.), alkoxycarbonyl, acylamino (for example benzyloxycarbonylamino etc.), acyloxy (for example acetoxy, benzyloxy etc.) and the like; preferred aliphatic acyl residues having such substituents which may be mentioned are alkanoyls substituted, for example, with amino, carboxy, amino and carboxy, halogen, acylamino or the like.

Aromatic acyl residues are deemed to comprise those acyl residue which originate from an acid with a substituted or unsubstituted aryl group, wherein the aryl group may comprise phenyl., toluyl, xylyl, naphthyl and the like; suitable examples are stated below:

aroyl (for example benzoyl, toluoyl, xyloyl, naphthoyl, phthaloyl etc.);
aralkanoyl (for example phenylacetyl etc.);
aralkenoyl (for example cinnamoyl etc.);
aryloxyalkanoyl (for example phenoxyacetyl etc.);
arylthioalkanoyl (for example phenylthioacetyl etc.);
arylaminoalkanoyl (for example N-phenylglycyl etc.);
arenesulfonyl (for example benzenesulfonyl, tosyl or toluenesulfonyl, naphthalenesulfonyl etc.);
aryloxycarbonyl (for example phenoxycarbonyl, naphthyloxycarbonyl etc.);
aralkoxycarbonyl (for example benzyloxycarbonyl etc.);
arylcarbamoyl (for example phenylcarbamoyl, naphthylcarbamoyl etc.);
arylglyoxyloyl (for example phenylglyoxyloyl etc.).

In the above examples of acyl residues, the aromatic hydrocarbon moiety (in particular the aryl residue) and/or the aliphatic hydrocarbon moiety (in particular the alkane residue) may optionally comprise one or more suitable substituents, such as those which have already been stated as suitable substituents for the alkyl group or the alkane residue. Aromatic acyl residues having particular substituents which may in particular be mentioned and constitute examples of preferred aromatic acyl residues are aroyl substituted with halogen and hydroxy or with halogen and acyloxy, and aralkanoyl substituted with hydroxy, hydroxyimino, dihaloalkanoyloxyimino, together with
arylthiocarbamoyl (for example phenylthiocarbamoyl etc.);
arylcarbamimidoyl (for example phenylcarbamimidoyl etc.).

A heterocyclic acyl residue is taken to mean an acyl residue which originates from an acid with a heterocyclic group; these include:

heterocyclic carbonyl, wherein the heterocyclic residue is an aromatic or aliphatic 5-to 6-membered heterocycle with at least one heteroatom from the group comprising nitrogen, oxygen and sulphur (for example thiophenyl, furoyl, pyrrolocarbonyl, nicotinoyl etc.);

alkanoyl heterocycle, wherein the heterocyclic residue is 5- to 6-membered and comprises at least one heteroatom from the group comprising nitrogen, oxygen and

sulphur (for example thiophenylacetyl, furylacetyl, imidazolylpropionyl, tetrazolylacetyl, 2-(2-amino-4-thiazolyl)-2-methoxyiminoacetyl etc.) and the like.

In the above examples of heterocyclic acyl residues, the heterocycle and/or the aliphatic hydrocarbon moiety may optionally comprise one or more suitable substituents, such as chose as have been stated to be suitable for alkyl and alkane groups.

"Alkyl groups" are straight- or branched-chain alkyl residues having 1 to 24 carbon atoms, such as methyl, ethyl, propyl, isopropyl, butyl, isobutyl, tert.-butyl, pentyl, hexyl and the like. They may be e.g. substituted with hydroxy, halogen or oxy groups.

Cycloalkyl preferably represents a optionally substituted C.sub.3-8-cycloalkyl; a. o. alkoxy (e.g. methoxy, ethoxy, etc.), halogen (e.g. fluorine, chlorine, bromine etc.), nitro and the like are suited to be possible substituents.

Aryl is an aromatic hydrocarbon residue, such as phenyl, naphthyl etc., which may optionally comprise one or more suitable substituents such as alkyl, alkoxy (for example methoxy, ethoxy etc.), trifluoromethylene, halogen (for example fluorine, chlorine, bromine etc.), nitro and the like.

"Aralkyl" includes mono-, di-, triphenylalkyl such as benzyl, phenethyl, benzhydryl, trityl and the like wherein the aromatic moiety may optionally comprise one or more suitable substituents such as alkoxy (for example methoxy, ethoxy etc.), halogen (for example fluorine, chlorine, bromine etc.), nitro and the like.

In the above ester the alkane and/or arene moiety may optionally comprise at least one suitable substituent, such as halogen, alkoxy, hydroxy, nitro and the like.

The invention further relates to the use of 3-N-formyl hydroxy amino propyl phosphonic acid derivatives and 3-N-acetyl hydroxy amino propyl phosphonic acid derivatives in combination with Piperaquine
and optionally
being selected from a second group consisting of
clindamycin, lincomycin, mirincamycin, pirlimycin and other lincosamides; minocycline and other tetracycline derivatives, or
azithromycin, erythromycin, spiramycin, josamycin, roxithromycin, clarithromycin, midecamycin and other macrolide antibiotics.'
or
artemisinin, Dihydroartemisinin
for therapeutic and prophylactic treatment of infections caused by bacteria, protozoa or uni- or multicellular parasites, preferably by uni- or multicellular parasites.

Hence, the inventive combinations can be used for the preparation of a medicament / drug, particularly for therapeutic and prophylactic treatment of infections caused by bacteria, protozoa or uni- or multicellular parasites, preferably by uni- or multicellular parasites.

In a preferred embodiment the inventive combinations can be used for therapeutic and prophylactic treatment of malaria, of babesiasis, of sleeping sickness, of Chagas' disease, of toxoplasmosis, of amoebic dysentery, of leishmaniases, of trichomoniasis, of pneumocystosis, of balantidiasis, of cryptosporidiosis, of sarcocytosis, of acanthamoebosis, of naeglerosis, of coccidiosis, of giardiasis and of lambliasis.

In a very preferred embodiment the inventive combinations can be used for therapeutic and prophylactic treatment of malaria and / or toxoplasmosis.

In a further preferred embodiment the inventive combinations can be used for bacterial and parasitic infections, particularly those that cause fever. Surprisingly, it could be found that the inventive combinations are highly suited for prophylaxis and treatment of bacterial and parasitic infections suffering / accompanying fever. Hence, in a preferred embodiment the invention relates to a pharmaceutical composition of the said combination for the syndromic treatment of fever and their use thereof. Moreover, the invention relates to a fever reducing agent comprising the combinations according to the invention.

The use of combination therapy with the help of pharmaceutical preparations of the present invention has the advantage of a synergistic increase of antiparasitic activity of the single substances as proved by the given examples. Hence, in combining the single compounds, there is a possibility of reducing the doses and, thus, the toxicity of the single compounds at the same time preserving antiparasitic activity. A combination therapy of the above listed principles of therapy of the individual compounds further provides the possibility of overcoming resistance and its use thereof.

With the use of said combination therapy it is possible to administer the active agents in a so-called fixed combination, i.e. in a single pharmaceutical formulation containing the active agents or to choose a so-called free combination, administering the active agents in form of separate pharmaceutical formulations at the same time or one after the other (e.g. in form of a blister).

If the active agents are solid materials, the active agents may be administered by conventional methods for solid drug preparations mixing e.g. all active agents and pelletizing them for example into pellets together with conventional excipients or auxiliary materials. However, it is also possible to provide the active agents separately in one package unit ready for sale wherein the package unit contains all active agents in separate pharmaceutical formulations.

The pharmaceutical preparations may be administered in liquid or solid form for enteral or parenteral application. In this connection all conventional forms of application are possible, for example pellets, capsules, dragees, sirups,

solutions, suspensions. Preferably, water is used as an injection medium containing added substances common in injection solutions such as stabilizers, dissolving intermediaries and buffers. If desired, preparations suited for oral application may contain flavorings or sweeteners.

The following example states the favourable activity of some representative combination preparations.

In the following, the present invention is described in more detail by way of examples. However, these examples are not intended to limit the scope of protection of the present invention in any way.

EXAMPLES

Study Objectives

The proposal covers in vitro drug combination studies between Fosmidomycin (FOS), Piperaquine (*PIP*) and Clindamycin (*CDN*) carried out in two steps. They will be conducted in accordance with international standards of GLP and UK standards of microbiological safety.

1. Initial determination of the in vitro efficacy profile (IC50/IC90) of FOS and PIP against a drug sensitive and a drug resistant strain of *Plasmodium falciparum. P. falciparum 3D7* is chloroquine sensitive, *P. falciparum K1* is insensitive to chloroquine

2. In vitro drug interactions of FOS in combination with PIP against a drug sensitive and a drug resistant strain of *P. falciparum* (3D7 and K1)

Materials & Methods

in vitro inhibition of parasite growth was assessed by the incorporation of [3H] hypoxanthine, including chloroquine (CQ) and artesunate (ASN) as positive control drugs. The activities of drug combinations were examined by the modified fixed ratio method (Canfield, C. J., M. Pudney, and W. E. Gutteridge. 1995. Interactions of atovaquone with other antimalarial drugs against Plasmodium falciparum in vitro. Exp Parasitol 80:373-81). In general, drug A + drug B solutions were freshly prepared in assay medium at ratios of 5:0, 4:1, 3:2, 2:3, 1:4 and 0:5, followed by 2-fold serial dilutions of each ratio, allowing the IC50 value to fall approximately at the midpoint of the serial dilution of each drug alone.

The first set of in vitro experiments established the IC50 values of the drugs alone, followed by Fixed Ratio Combination assays of FOS+PIP with FOS+CND as a control combination. Chloroquine and artesunate were included in all assays to ensure the validity of the test.

Drugs

Micronised Fosmidomycin (FOS) (Jomaa Pharma GmbH, Hamburg) was dissolved in distilled water at 10mg/ml

Piperaquine phosphate (PIP) (Prof R Hayes, University of Hong Kong) was dissolved in distilled water at 10mg/ml

Clindamycin hydrochloride (CND) (Sigma, UK) was dissolved in DMSO at 10mg/ml (Batch No. 125K1133)

Chloroquine diphosphate (CQ) (Sigma, UK) was dissolved in distilled water at 10mg/ml (Cat No. 25745 Lot & filling No. 1335410 43207097)

Artesunate (ASN) (Bayer AG, Leverkusen, Germany) was dissolved in DMSO at 10mg/ml (Cat No. DHN060302)

IC50 values were used to calculate FIC50/90 values for each drug ratio (FIC = Fractional Inhibitory Concentration) as previously described (Berenbaum, M. C. 1978. A method for testing for synergy with any number of agents. J Infect Dis 137:122-30, Borrmann, S., I. Lundgren, S. Oyakhirome, B. Impouma, P. B. Matsiegui, A. A. Adegnika, S. Issifou, J. F. Kun, D. Hutchinson, J. Wiesner, H. Jomaa, and P. G. Kremsner. 2006. Fosmidomycin plus clindamycin for treatment of pediatric patients aged 1 to 14 years with Plasmodium falciparum malaria. Antimicrob Agents Chemother 50:2713-8. Canfield, C. J., M. Pudney, and W. E. Gutteridge. 1995. Interactions of atovaquone with other antimalarial drugs against Plasmodium falciparum in vitro. Exp Parasitol 80:373-81). ∑FIC50/90 values of PIP and ASN were calculated by:

$$\Sigma FIC_{50/90} = \frac{IC_{50/90}\text{ of drug A in combination}}{IC_{50/90}\text{ of drug A alone}} + \frac{IC_{50/90}\text{ of drug B in combination}}{IC_{50/90}\text{ of drug B alone}}$$

An overall mean ∑FIC50 or ∑FIC90 value for each combination is determined and synergy or antagonism

defined as a mean ∑FIC ≤ 0.5 or > 4 respectively (Berenbaum, supra).

The first sets of experiments were carried out to establish the IC50 and IC90 values of the drugs on their own. The drugs were evaluated in 3 separate experiments (see Tables 1 & 2)

Table 1: IC50 values of drugs against P. falciparum 3D7 and K1 IC50 μg/ml

| **Experiment** | | **1** | **2** | **3** |
|---|---|---|---|---|
| **FOS** | 3D7 | 0.18 | 0.23 | 0.09 |
| | K1 | - * | -* | 0.17 |
| **CDN** | 3D7 | <0.21 | <0.21 | 0.26 |
| | K1 | 46.19 | 45.15 | 0.53 |
| **PIP** | 3D7 | 0.01 | 0.006 | 0.01 |
| | K1 | 0.02 | 0.02 | 0.01 |
| **ASN** | 3D7 | 0.0019 | 0.002 | 0.0008 |
| | K1 | 0.005 | 0.002 | 0.002 |

*Experiment failed

Table 2: IC90 values of drugs against P. falciparum 3D7 and K1

| **Experiment** | | **1** | **2** | **3** |
|---|---|---|---|---|
| **FOS** | 3D7 | 0.93 | 0.44 | 0.62 |
| | K1 | -* | -* | 0.41 |
| **CDN** | 3D7 | 0.27 | 34.08 | >10 |
| | K1 | >50 | >50 | >50 |
| **PIP** | 3D7 | 0.018 | 0.02 | 0.01 |
| | K1 | 0.06 | 0.05 | 0.02 |
| **ASN** | 3D7 | 0.007 | 0.002 | 0.002 |
| | K1 | 0.006 | 0.003 | 0.004 |

*Experiment failed

($IC_{99}$ values can be found in the Appendix)

Subsequently, the combination of FOS + PIP was evaluated against both parasite strains in two separate experiments (Table 3)

Table 3: Mean ∑FIC values of FOS + PIP against P. falciparum 3D7 and K1

| | FOS + PIP | | FOS + CND (Control) |
|---|---|---|---|
| | **∑FIC$_{50}$** | | |
| **3D7** | 1.46 | 1.27 | 0.49 |
| **K1** | 1.26 | 1.29 | 0.63 |
| | **∑FIC**$_{90}$ | | |
| **3D7** | 1.27 | 1.25 | 0.49 |
| **K1** | -* | 1.1 | -* |
| *IC$_{90}$ could not be determined | | | |

The in vitro combination studies confirmed the synergistic interaction between FOS plus CDN (see Table 3) and this data is similar to that already reported (Wiesner, J., D. Henschker, D. B. Hutchinson, E. Beck, and H. Jomaa. 2002. In vitro and in vivo synergy of fosmidomycin, a novel antimalarial drug, with clindamycin. Antimicrob Agents Chemother 46:2889-94). The combination of FOS plus PIP showed significantly an additive effect (Table 3), with FIC values in the range of 1.1 to 1.5, when analysed using both IC50 and IC90 values in two separate experiments. There was no significant difference in results against the CQ-sensitive strain and the K1 CQ-resistant of *P. falciparum.* Hence, these experiments indicate a synergetic effect of the inventive FOS plus PIP combination.

## Claims

1. Pharmaceutical composition comprising a compound of the formula

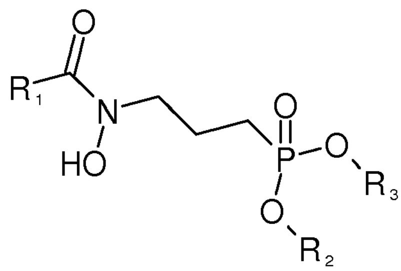

wherein R.sub.1 is selected from the group consisting of hydrogen and methyl, and
wherein R.sub.2 and R.sub.3 are independently selected from the group, consisting of hydrogen, substituted or unsubstituted alkyl, substituted or unsubstituted acyl, substituted or unsubstituted aryl, substituted or unsubstituted aralkyl, substituted or unsubstituted cycloalkyl, substituted or unsubstituted silyl, substituted or unsubstituted heterocyclic residue, or together form a substituted or unsubstituted C.sub.1-5-alkyl chain, the alkyl groups being saturated or comprising one or more double bonds or triple bonds,
and in combination with
Piperaquine
and, if appropriate, a salt thereof and, if required, further adjuvants.

2. Pharmaceutical composition in accordance with claim 1 comprising a further active ingredient
being selected from a second group consisting of
clindamycin, lincomycin, mirincamycin, pirlimycin and other lincosamides; minocycline and other tetracycline derivatives,
or
azithromycin, erythromycin, spiramycin, josamycin, roxithromycin, clarithromycin, midecamycin and other macrolide antibiotics.'
or
artemisinin, Dihydroartemisinin
and, if appropriate, a salt thereof and, if required, further adjuvants.

3. Pharmaceutical composition in accordance with claim 1 or claim 2, wherein the formula I represents fosmydomycin or a salt thereof.

4. Pharmaceutical composition in accordance with claim 2, wherein the third compound is clindamycin or azithromycin or a salt thereof.

5. Pharmaceutical composition in accordance with claim 2, wherein the third compound is artemisinin or Dihydroartemisinin or a salt thereof.

6. Pharmaceutical composition in accordance with claims 1 to 5 for therapeutic and prophylactic treatment of infections caused by bacteria, protozoa or uni- or multicellular parasites, preferably by uni- or multicellular parasites.

7. Pharmaceutical composition in accordance with claims 1 to 5 for therapeutic and prophylactic treatment of malaria, of babesiasis, of sleeping sickness, of Chagas' disease, of toxoplasmosis, of amoebic dysentery, of leishmaniases, of trichomoniasis, of pneumocystosis, of balantidiasis, of cryptosporidiosis, of sarcocytosis, of acanthamoebosis, of naeglerosis, of coccidiosis, of giardiasis and of lambliasis.

8. Pharmaceutical composition in accordance with claims 1 to 5 for therapeutic and prophylactic treatment of malaria and / or toxoplasmosis.

9. Pharmaceutical composition in accordance with claims 1 to 5 being a fever reducing agent.

10. Use of a pharmaceutical composition of one of claims 1 to 5 for therapeutic and prophylactic treatment of infections caused by bacteria and parasites, particularly protozoa or uni- or multicellular parasites.

11. Use of a pharmaceutical composition of one of claims 1 to 5 for the syndromic treatment of fever.

12. Use of a pharmaceutical composition of one of claims 1 to 5 as a fever reducing agent.

13. Use according to claims 10 to 12 for therapeutic and prophylactic treatment of malaria, of babesiasis, of sleeping sickness, of Chagas' disease, of toxoplasmosis, of amoebic dysentery, of leishmaniases, of trichomoniasis, of pneumocystosis, of balantidiasis, of cryptosporidiosis, of sarcocytosis, of acanthamoebosis, of naeglerosis, of coccidiosis, of giardiasis and of lambliasis.

14. Use according to claim 10 for therapeutic and prophylactic treatment of malaria and / or toxoplasmosis.

Europäisches Patentamt
European Patent Office
Office européen des brevets

# EUROPEAN SEARCH REPORT

Application Number
EP 10 16 8641

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2001 081034 A (FUJISAWA PHARMACEUTICAL CO) 27 March 2001 (2001-03-27)<br>* the whole document *<br>----- | 1-14 | INV.<br>A61K31/496<br>A61K31/664<br>A61P33/00<br>A61P33/06 |
| Y,D | EP 1 305 031 B1 (JOMAA PHARMAKA GMBH [DE] BIOAGENCY AG [DE])<br>14 May 2008 (2008-05-14)<br>* claims 1-7 *<br>----- | 1-14 | |
| Y | WO 2010/004573 A1 (IPCA LAB LTD [IN]; PAREEK ANIL [IN])<br>14 January 2010 (2010-01-14)<br>* claims 1, 3 *<br>----- | 1-14 | |
| Y | YOKOTA Y ET AL: "IN VITRO SYNERGISM OF FR-31564, A NEW PHOSPHONIC ACID ANTIBIOTIC",<br>JOURNAL OF ANTIBIOTICS, JAPAN ANTIBIOTICS RESEARCH ASSOCIATION, TOKYO, JP,<br>vol. 34, no. 7, 1 July 1981 (1981-07-01), pages 876-883, XP000867644,<br>ISSN: 0021-8820<br>* page 877, paragraph 1 *<br>----- | 1-14 | TECHNICAL FIELDS SEARCHED (IPC)<br>A61K |
| Y | SNYDER ET AL: "In vitro and in vivo interaction of synthetic peroxide RBx11160 (OZ277) with piperaquine in Plasmodium models",<br>EXPERIMENTAL PARASITOLOGY, NEW YORK, NY, US,<br>vol. 115, no. 3,<br>2 December 2006 (2006-12-02), pages 296-300, XP005724230,<br>ISSN: 0014-4894, DOI: DOI:10.1016/J.EXPPARA.2006.09.016<br>* abstract *<br>-----<br>-/-- | 1-14 | |

1

The present search report has been drawn up for all claims

| Place of search | Date of completion of the search | Examiner |
|---|---|---|
| Munich | 19 November 2010 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 16 8641

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | BERENBAUM M C: "A method for testing for synergy with any number of agents", JOURNAL OF INFECTIOUS DISEASES, UNIVERSITY OF CHICAGO PRESS, CHICAGO, IL, vol. 137, no. 2, 1 January 1978 (1978-01-01), pages 122-130, XP008082150, ISSN: 0022-1899 <br>* page 122, left-hand column, last paragraph - right-hand column, paragraph 1 * <br>* page 123, left-hand column, paragraph 3 * | 1-14 | |
| Y | ARAV-BOGER RAVIT ET AL: "Molecular mechanisms of resistance in antimalarial chemotherapy: the unmet challenge.", ANNUAL REVIEW OF PHARMACOLOGY AND TOXICOLOGY 2005 LNKD- PUBMED:15822189, vol. 45, 2005, pages 565-585, XP002610469, ISSN: 0362-1642 <br>* page 576, last paragraph * | 1-14 | TECHNICAL FIELDS SEARCHED (IPC) |

The present search report has been drawn up for all claims

1

| Place of search | Date of completion of the search | Examiner |
|---|---|---|
| Munich | 19 November 2010 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 10 16 8641

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2010

| Patent document cited in search report | | Publication date | Patent family member(s) | | | Publication date |
|---|---|---|---|---|---|---|
| JP 2001081034 | A | 27-03-2001 | NONE | | | |
| EP 1305031 | B1 | 14-05-2008 | AT | 395064 | T | 15-05-2008 |
| | | | AU | 6756101 | A | 08-01-2002 |
| | | | CA | 2420640 | A1 | 26-02-2003 |
| | | | DE | 10030781 | A1 | 17-01-2002 |
| | | | WO | 0200208 | A2 | 03-01-2002 |
| | | | EP | 1305031 | A2 | 02-05-2003 |
| | | | ES | 2306718 | T3 | 16-11-2008 |
| | | | US | 2004235784 | A1 | 25-11-2004 |
| WO 2010004573 | A1 | 14-01-2010 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 19825585
- DE 2733658
- EP 1071409 B1
- EP 1305031 A


### Non-patent literature cited in the description


- **JOMAA, H. ; J. WIESNER ; S. SANDERBRAND ; B. ALTINCICEK ; C. WEIDEMEYER ; M. HINTZ ; I. TURBACHOVA ; M. EBERL ; J. ZEIDLER ; H. K. LICHTENTHALER.** Inhibitors of the nonmevalonate pathway of isoprenoid biosynthesis as antimalarial drugs. *Science,* 1999, vol. 285, 1573-6
- **LELL, B. ; R. RUANGWEERAYUT ; J. WIESNER ; M. A. MISSINOU ; A. SCHINDLER ; T. BARANEK ; M. HINTZ ; D. HUTCHINSON ; H. JOMAA ; P. G. KREMSNER.** Fosmidomycin, a novel chemotherapeutic agent for malaria. *Antimicrob Agents Chemother,* 2003, vol. 47, 735-8
- **BORRMANN, S. ; A. A. ADEGNIKA ; F. MOUSSAVOU ; S. OYAKHIROME ; G. ESSER ; P. B. MATSIEGUI ; M. RAMHARTER ; 1. LUNDGREN ; M. KOMBILA ; S. ISSIFOU.** Short-course regimens of artesunate-fosmidomycin in treatment of uncomplicated Plasmodium falciparum malaria. *Antimicrob Agents Chemother,* 2005, vol. 49, 3749-54
- **BORRMANN, S. ; S. ISSIFOU ; G. ESSER ; A. A. ADEGNIKA ; M. RAMHARTER ; P. B. MATSIEGUI ; S. OYAKHIROME ; D. P. MAWILI-MBOUMBA ; M. A. MISSINOU ; J. F. KUN.** Fosmidomycin-clindamycin for the treatment of Plasmodium falciparum malaria. *J Infect Dis,* 2004, vol. 190, 1534-40
- **NOEDL, H. ; Y. SE ; K. SCHAECHER ; B. L. SMITH ; D. SOCHEAT ; M. M. FUKUDA.** Evidence of artemisinin-resistant malaria in western Cambodia. *N Engl J Med,* 2008, vol. 359, 2619-20
- **CANFIELD, C. J. ; M. PUDNEY ; W. E. GUTTERIDGE.** Interactions of atovaquone with other antimalarial drugs against Plasmodium falciparum in vitro. *Exp Parasitol,* 1995, vol. 80, 373-81
- **BERENBAUM, M. C.** A method for testing for synergy with any number of agents. *J Infect Dis,* 1978, vol. 137, 122-30
- **BORRMANN, S. ; I. LUNDGREN ; S. OYAKHIROME ; B. IMPOUMA ; P. B. MATSIEGUI ; A. A. ADEGNIKA ; S. ISSIFOU ; J. F. KUN ; D. HUTCHINSON ; J. WIESNER.** Fosmidomycin plus clindamycin for treatment of pediatric patients aged 1 to 14 years with Plasmodium falciparum malaria. *Antimicrob Agents Chemother,* 2006, vol. 50, 2713-8
- **WIESNER, J. ; D. HENSCHKER ; D. B. HUTCHINSON ; E. BECK ; H. JOMAA.** In vitro and in vivo synergy of fosmidomycin, a novel antimalarial drug, with clindamycin. *Antimicrob Agents Chemother,* 2002, vol. 46, 2889-94